(19) Europäisches Patentamt
European Patent Office
Office européen des brevets

(11) Veröffentlichungsnummer: **0 083 008**
**B1**

(12) # EUROPÄISCHE PATENTSCHRIFT

(45) Veröffentlichungstag der Patentschrift: **02.10.85**

(21) Anmeldenummer: **82111516.9**

(22) Anmeldetag: **11.12.82**

(51) Int. Cl.⁴: **C 09 K 15/30**, C 07 C 97/07, C 07 C 101/04, C 07 D 265/36, C 09 K 15/24

(54) **Aminoreduktone und mit Aminoreduktonen als Antioxidantien stabilisierte organische Materialien.**

(30) Priorität: **28.12.81 DE 3151534**

(43) Veröffentlichungstag der Anmeldung: **06.07.83 Patentblatt 83/27**

(45) Bekanntmachung des Hinweises auf die Patenterteilung: **02.10.85 Patentblatt 85/40**

(84) Benannte Vertragsstaaten: **BE CH DE FR GB IT LI NL**

(56) Entgegenhaltungen:
**US - A - 3 816 137**

**CHEMICAL ABSTRACTS, Band 95, Nr. 9, 31. August 1981, Seite 721, Nr. 80249n, Columbus, Ohio, USA K. SCHANK et al.: "Introduction of oxygen function into the alpha-position of beta-diketones. 5. Acyloxylation of 3-primary-amino-2-cyclohexen-1-on es.**
**CHEMICAL ABSTRACTS, Band 91, Nr. 9, 27. August 1979, Nr. 74550e, Columbus, Ohio, USA M. ADLER et al.: "Introduction of oxygen functional groups into the alpha-position of beta-diketones. 2. Defunctionalizations of 2-(acyloxy)-3-(secondary amino)-2-cyclohexen-1-ones.**

(73) Patentinhaber: **BASF Aktiengesellschaft, Carl-Bosch-Strasse 38, D-6700 Ludwigshafen (DE)**

(72) Erfinder: **Lechtken, Peter, Dr., Ludwigshafener Strasse 6b, D-6710 Frankenthal (DE)**
Erfinder: **Mueller, Gerhard, Dr., Maxburgerstrasse 10, D-6728 Germersheim (DE)**
Erfinder: **Schmieder, Klaus, Dr., Sonnenstrasse 12 b, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 083 008 B1

**Beschreibung**

Die vorliegende Erfindung betrifft organische Materialien, die 0,001—10 Gew.-% eines Aminoreduktons der allgemeinen Formel I

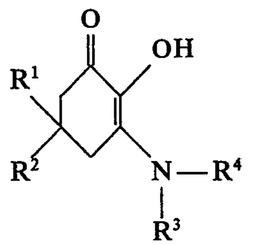

(I)

oder eines mineralsauren Salzes davon als Antioxidans enthalten.

In dieser Formel bedeuten:

$R^1$, $R^2$, $R^3$ H; die Methylgruppe oder die Ethylgruppe

$R^4$ den an eine primäre Aminogruppe gebundenen Rest einer natürlichen $\alpha$- oder $\beta$-Aminosäure oder einen $C_1-C_{10}$-Alkylester davon oder, falls es sich um eine $\alpha$-Aminosäure handelt um einen Rest, in welchem die Carboxylgruppe der Aminosäure und die 2-Hydroxylgruppe des Cyclohexenonrings ein Lacton bilden;
eine $C_1-C_{20}$-Alkyl- oder Alkenylgruppe, die als Substituenten eine oder mehrere Hydroxylgruppen, $C_1-C_4$-Alkoxygruppen oder $C_1-C_4$-Acyloxygruppen tragen kann, oder
einen Rest $-(CH_2-CH_2-O)_nH$, der mit einem $C_1-C_4$-Alkanol verethert oder mit einer $C_1-C_4$-Fettsäure verestert sein kann, wobei n für 2—10 steht.

Ferner betrifft die Erfindung die Verwendung der Aminoreduktone I zum Stabilisieren organischer Materialien gegen oxidative Einflüsse sowie die Herstellung dieser Verbindungen und die neuen Aminoreduktone der allgemeinen Formel Ia

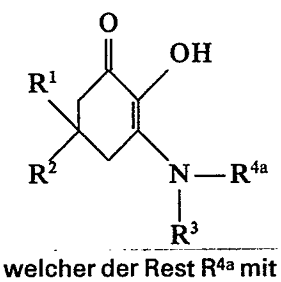

(Ia)

in welcher der Rest $R^{4a}$ mit Ausnahme der Methylgruppe die Bedeutung von $R^4$ hat.

Unter Reduktonen versteht man im allgemeineren Sinne stark reduzierende organische Substanzen, darunter hauptsächlich solche, die sich von Zuckern ableiten. Im engeren Sinne bezeichnet man als Reduktone Verbindungen mit dem Strukturelement

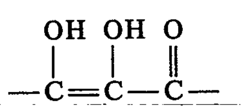

wobei die Hydroxylgruppen und der Sauerstoff der Oxogruppe auch durch die entsprechenden Aminfunktionen ersetzt sein können.

Die Reduktone — sowohl im weiteren als auch im engeren Sinne — wurden verschiedentlich schon als Antioxydantien für organische Materialien, insbesondere für Lebensmittel, vorgeschlagen, zeigen in dieser Eigenschaft jedoch uneinheitliche und darüber hinaus sogar unerwünschte Wirkungen, indem sie die Qualität, insbesondere die Geschmacksqualität der Lebensmittel, beeinträchtigen.

So untersuchten Obata et al. (Nippon Nogei Kagaku Kaishi Band 45 (11), 1971, Seiten 489—495; C. A. 77, 1972, Ref. No. 3944 m) Ascorbinsäure (L-3,4-Dihydroxy-5-(1,2-dihydroxyethyl)-2,5-dihydrofuran-2-on), Triosereadukton (2,3-Dihydroxyacrolein), 2,3-Dihydroxy-pent-2-en-1-on und einige Aminosäurederivate des Triosereduktons

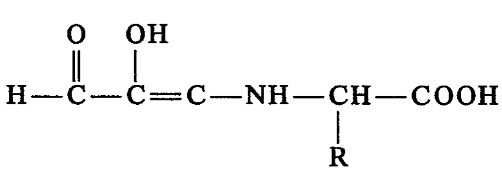

R = H; Methyl; Propyl-, Butyl-, Mercaptopropyl

sowie in einer weiteren Arbeit (Nippon Nogei Kagaku Kaishi, Band 54 (7), 1980, Seiten 542—544, C. A. 93, 1980, Ref. No. 166 211q) verschiedene Anile des Triosereduktons.

Während die Aminosäurederivate des Triosereduktons bei Sojabohnenöl und Baumwollsamenöl eine bessere antioxidative Wirkung zeigen als eines der gebräuchlichsten Standard-Antioxidantien, nämlich einer Mischung aus 2- und 3-tert.-Butyl-4-hydroxyanisol (»BHA«), sind die Anile im Falle der Stabilisierung von Schweineschmalz deutlich schlechter geeignet als BHA.

Insgesamt ist den beiden Arbeiten zu entnehmen, daß die unsubstituierten Reduktone wie Triosereduktron, Ascorbinsäure und 2,3-Dihyroxy-pent-2-en-1-on als Antioxidantien kaum in Betracht kommen und daß auch die Amino- und Iminoderivate dieser Verbindungen in dieser Eigenschaft erheblich zu wünschen übrig lassen. Davon abgesehen, bereitet die Herstellung dieser höchst empfindlichen Verbindungen große präparative Schwierigkeiten.

Aus der US-PS 3 816 137 sind bestimmte 3-Aminoderivate des 2,3-Dihydroxy-cyclohex-2-en-1-ons als photographische Entwickler bekannt. Diese Aminoderivate leiten sich vom Methylamin, vom Dimethylamin sowie hauptsächlich von den heterocyclischen Aminen Piperidin und Morpholin ab. Die Herstellung dieser Reduktone erfolgt durch Umsetzung des betreffenden Amins mit 3-Chlor-cyclohexan-1,2-dion in Gegenwart von Triethylamin. Dieses Verfahren, welches von H. Simon et al. näher beschrieben ist (Chem. Ber. 98, 1965, Seiten 3692—3702), ist jedoch wegen der schlechten Zugänglichkeit der 3-Chlorcyclohexan-1,2-dione für technische Zwecke wenig geeignet und verläuft mit primären Aminen außerdem nicht einheitlich.

Aus der Arbeit von Cocker et al. (J. Chem. Soc. 1950, Seiten 2052—2058) ist es weiterhin bekannt, Triosereduktron mit aromatischen Aminen sowie mit $\alpha$-Aminosäuren zu den entsprechenden 3-Substitutionsprodukten des 2,3-Dihydroxyacroleins umzusetzen. Im Falle der Aminosäuren sind die Ausbeuten mit etwa 10—36%, bezogen auf das Triosereduktron, jedoch unbefriedigend.

Der Erfindung lag die Aufgabe zugrunde, organische Materialien wirksamer als bisher gegen oxidative Einflüsse zu stabilisieren und hierfür größtenteils neue und allgemein gut zugängliche physiologisch unbedenkliche Antioxidantien bereitzustellen.

Demgemäß wurde gefunden, daß organische Materialien, die einen Gehalt von 0,001—10 Gew.-% der eingangs definierten Aminoreduktone enthalten, hervorragend gegen oxidative Einflüsse stabiliert sind.

Weiterhin wurde gefunden, daß man die Aminoreduktone I in einer bemerkenswert glatten Reaktion sowie in hohen Ausbeuten durch Umsetzung eines 2,3-Dihydroxycyclohex-2-en-1-ons der allgemeinen Formel II

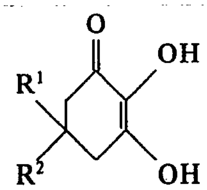

(II)

mit einer Aminoverbindung der allgemeinen Formel III

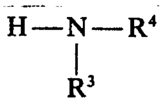

(III)

oder einem mineralsauren Salz davon erhält.

Die Ausgangsverbindungen II, unter denen diejenigen bevorzugt werden, in denen beide Reste $R^1$ und $R^2$ Wasserstoff bedeuten, sind bekannt oder nach bekannten Methoden erhältlich, z. B. nach dem in Houben-Weyl, Methoden der organischen Chemie, Band 6/1d, Seite 266 beschriebenen Verfahren durch Hydrierung von Pyrogallol in alkalischer Lösung.

Als Aminoverbindungen III kommen vor allem diejenigen in Betracht, in denen $R^3$ Wasserstoff bedeutet und in denen sich $R^4$ von einer natürlichen $\alpha$- oder $\beta$-Aminosäure mit mindestens einer primären Aminogruppe ableitet. Unter dem Begriff natürliche Aminosäuren sind, wie allgemein üblich, diejenigen zu verstehen, die in der Natur als Bausteine insbesondere von Proteinen oder als Stoffwechselprodukte vorkommen. Diese Säuren und auch deren $C_1-C_{10}$-Alkylester sind physiologisch unbedenklich und bedingen daher die physiologische Unbedenklichkeit der Verbindungen I, die beim Stoffwechsel zunächst zu III und den nach bisherigen Feststellungen ebenfalls unbedenklichen Reduktonen II oder Oxidationsprodukten hier abgebaut werden können.

Derartige Säuren, bei denen die Konfiguration im vorliegenden Zusammenhang keine Rolle spielt (d. h. man kann sie in ihren optischen aktiven Formen oder, was meist wirtschaftlicher ist, in Form ihrer

Racemate einsetzen) sind beispielsweise

Glycin (Gly)

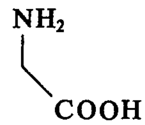

Alanin (Ala)

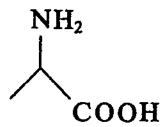

Valin (Val)

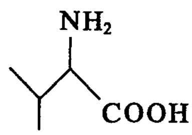

Leucin (Leu)

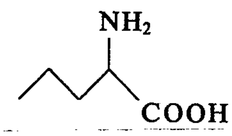

Isoleucin (Ileu)

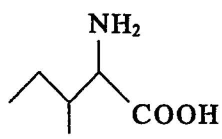

Phenylalanin (Phe)

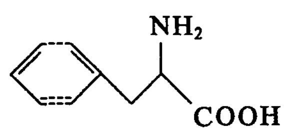

Tyrosin (Tyr)

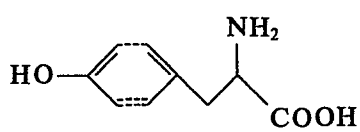

Serin (Ser)

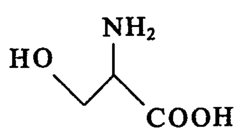

Threonin (Thr)

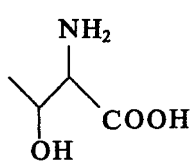

Asparaginsäure (Asp)

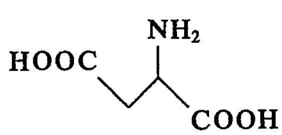

Glutaminsäure (Glu)

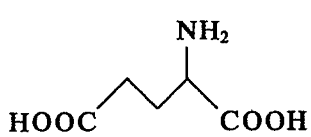

Lysin (Lys)

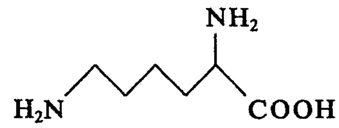

β-Alanin

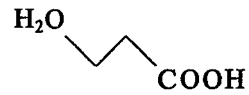

Die Umsetzungsprodukte von II mit den α-Aminosäuren sind entweder die entsprechenden freien Säuren Ia oder deren Lactone Ib

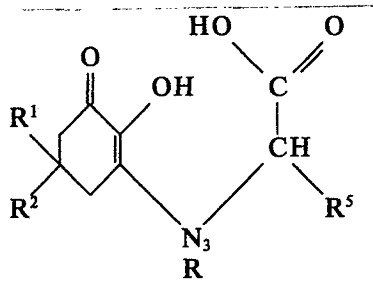

(Ia)

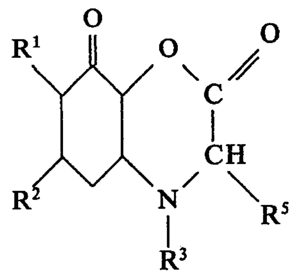

(Ib)

$R^5$ = charakteristischer Rest der definitionsgemäßen α-Aminosäuren

Da es sich bei den zu stabilisierenden organischen Materialien meist um organophile hydrophobe Substanzen wie Öle und Fette handelt, ist es zweckmäßig, von den Estern dieser Säuren auszugehen, denn die entsprechenden Verbindungen I sind in diesem Falle mit organischen Materialien verträglicher und können leichter in diese eingearbeitet werden.

Als Esterreste kommen alle diejenigen in Betracht, die sich von unverzweigten oder verzweigten $C_1-C_{10}$-Alkanolen ableiten, wobei sich die Organophilie allgemein und besonders die Fettlöslichkeit mit steigendem C-Gehalt erhöhen.

Eine weitere Gruppe von geeigneten Aminoverbindungen III sind die $C_1-C_{20}$-Alkyl- und Alkenylamine und deren N-Methyl- und N-Ethylderivate.

Als Verbindungen dieser Art kommen beispielsweise in Betracht:

Methylamin
Dimethylamin
Ethylamin
Ethylmethylamin
Diethylamin
Propylamin
Isopropylamin
Butylamin
Hexylamin
2-Ethylhexylamin
Dodecylamin
Palmitylamin und
Stearylamin.

Diese und die weiteren definitionsgemäßen Amine können als Substituenten Hydroxylgruppen enthalten, die ihrerseits mittels eines $C_1-C_4$-Alkanols oder einer $C_1-C_4$-Fettsäure verethert bzw. verestert sein können. Vorzugsweise befinden sich die Hydroxylgruppe bzw. die Alkoxygruppe oder die Acyloxygruppe in 2-Stellung zur Aminogruppe, da die entsprechenden Alkanolamine, beispielsweise

Ethanolamin
Aminopropan-2-ol
N-Methylethanolamin
Aminobutan-2-ol
Aminohexan-2-ol und
Aminododecan-2-ol

leicht durch Umsetzung von den entsprechenden 1,2-Epoxyverbindungen, die ihrerseits aus den entsprechenden α-Olefinen erhältlich sind, mit Ammoniak, Methylamin oder Ethylamin hergestellt werden können.

Amine III, welche in den Aminoreduktonen I besonders gute anwendungstechnische Eigenschaften bedingen, sind solche, die durch Oxethylierung von Ammoniak, Methylamin oder Ethylamin erhältlich sind, also beispielsweise

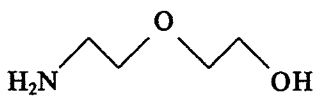

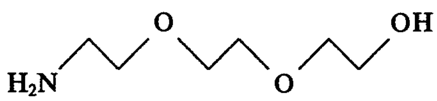

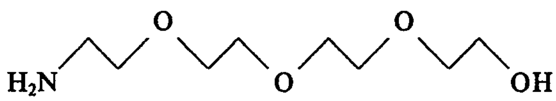

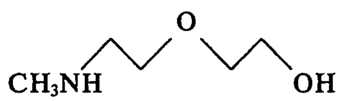

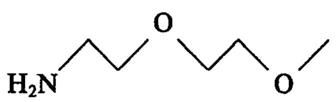

und

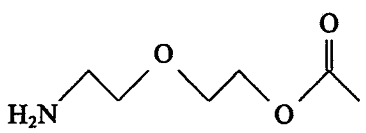

Die entsprechenden Verbindungen I sind wasserlöslich oder selbstdispergierend und können daher in Form ihrer wäßrigen Lösungen bzw. Dispersionen in flüssige organische Materialien und deren Zubereitungen, z. B. Vitaminzubereitungen, eingearbeitet werden.

Zur Herstellung der Aminoreduktone I setzt man 1 mol II mit 1—3 mol, vorzugsweise mit äquimolaren oder annähernd äquimolaren Mengen III entweder in Substanz oder in Gegenwart einer vorzugsweise aprotischen Flüssigkeit wie Toluol oder Xylol, in welcher die Reaktionspartner nicht gelöst zu sein brauchen, bei etwa 30—150, besonders bei 50—120°C miteinander um. Die Gegenwart von Wasser stört in einigen Fällen nicht, und in den anderen wird das Wasser ohnehin zweckmäßigerweise zusammen mit dem entstehenden Wasser im Laufe der Umsetzung entfernt.

Sofern eine α-Aminosäure eingesetzt wird, erhält man entweder das offenkettige Derivat Ia von II oder das zum Lacton cyclisierte Derivat Ib. Ist Wasser zugegen, entsteht vorwiegend das offenkettige Derivat Ia, und wird das Wasser entfernt, bildet sich meistens das Lacton Ib. Häufig erhält man Mischungen von Ia und Ib, die in dieser Form verwendet werden können, da beide Verbindungen hinsichtlich ihrer antioxidativen Wirkung gleichwertig sind.

Es empfiehlt sich, die Umsetzung durch Mitverwendung katalytischer Mengen einer Säure wie Chlorwasserstoff, p-Toluolsulfonsäure oder eines sauren Ionenaustauschers zu beschleunigen, jedoch gelingt die Reaktion auch ohne Säure.

Da es sich sowohl bei I als auch bei II um höchst oxidationsempfindliche Verbindungen handelt, versteht es sich von selbst, unter strengem Ausschluß von Luftsauerstoff zu arbeiten, etwa indem man alle Operationen unter Stickstoff vornimmt.

Die Aufarbeitung auf die Verfahrensprodukte I erfolgt wie üblich, so daß sich nähere Angaben hierzu erübrigen.

Die Verfahrensprodukte I, die man auch auf anderem Wege, wenn auch nicht so vorteilhaft, herstellen kann, z. B. aus den 3-Chlor-2-hydroxy-cyclohex-2-en-1-onen und den Aminoverbindungen III, sind im Rahmen der Definition des Restes $R^{4a}$ entsprechend der Stoffgruppe Ia neu.

Unter diesen neuen Verbindungen werden diejenigen bevorzugt, in denen $R^1$ und $R^2$ Wassertoff und $R^3$ Wasserstoff oder die Methylgruppe bedeuten. Hinsichtlich des Restes $R^{4a}$ kommen besonders die einfachen Aminosäuren Gly, Ala, Val, Len, Ser, Thr und β-Alanin, die $C_2-C_{10}$-Alkylamine und deren 2-Hydroxyderivate sowie die Amine des Polyethertyps in Betracht.

Organische Materialien, die sich mittels der Verbindung I erfolgreich gegen oxidative Einflüsse

stabilisieren lassen, sind vor allem Lebens-, Arznei- und Futtermittel aller Art, darunter besonders Fruchtsäfte, Öle und Fette sowie Öl- und fetthaltige Zubereitungen. Besondere Bedeutung haben die Antioxidantien für die Stabilisierung von mehrfach zu verwendenden Fetten (Fritierfetten) und von teuren hochempfindlichen Substanzen wie Vitamin A und anderen Verbindungen der Carotinoidreihe oder von wertvollen oxidationsempfindlichen Duft- und Aromastoffen.

Weiterhin seien als Substrate Kunststoffe genannt, die mit Lebensmitteln in Berührung kommen.

Die Konzentration der Antioxidantien richtet sich nach den Anforderungen an den Stabilisierungsgrad. Sie liegt allgemein zwischen etwa 0,001 und 10 Gew.-%. Wird das Substrat keinen extremen Einflüssen ausgesetzt und ist es zum baldigen Verbrauch bestimmt, genügen Konzentrationen zwischen 0,001 und 0,05 Gew.-%, handelt es sich beispielsweise um Fritierfette, liegen die Konzentrationen etwa zwischen 0,01 und 1,0 Gew.-%, und lediglich bei sehr empfindlichen und teuren Präparaten wie Vitaminen, bei denen mit längeren Lagerzeiten gerechnet werden muß, können sich Konzentrationen bis zu 10 Gew.-% empfehlen.

Die Einarbeitung der erfindungsgemäßen Antioxidantien in die organischen Materialien kann wie üblich vorgenommen werden, so daß nähere Ausführungen hierzu entbehrlich sind.

Es ist bemerkenswert, daß die erfindungsgemäßen Aminoreduktone einerseits in ihrer antioxidativen Funktion hochwirksam sind, andererseits aber nicht so reaktiv, daß sie, wie die meisten anderen Verbindungen vom Reduktontyp, nach den bisherigen Beobachtungen nicht zu anderweitigen Reaktionen oder zur Selbstzersetzung neigen.

## Beispiele

Im folgenden wird der Rest

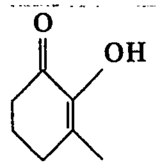

als Rest A bezeichnet. Alle Operationen wurden unter Stickstoff ausgeführt.

### Beispiel 1

$A-NH-CH_2-COOH$

Eine Lösung aus 150 g (2 mol) Glycin und 450 ml Wasser wurde unter Rühren mit 106,5 g (0,83 mol) A—OH und 75 ml 2 n Salzsäure versetzt und zum Sieden erhitzt. Bei etwa 40°C entstand eine klare Lösung, aus der bei ungefähr 80°C das Verfahrensprodukt auszufallen begann. Nach Erreichen der Siedetemperatur wurde das Gemisch auf 10°C abgekühlt und wie üblich auf den Niederschlag, ein gelbliches Kristallpulver, aufgearbeitet. Umkristallisation aus heißem Isopropanol lieferte das oben genannte Aminoredukton in 88%iger Ausbeute in Form farbloser Kristalle, Smp. 185—187°C.

### Beispiel 2

$A-NH-CH_2-COO-C_2H_5$

Eine Mischung aus 128 g (1 mol) A—OH, 149,5 g (1 mol) Glycinethylesterhydrochlorid, 84 g (1 mol) $NaHCO_3$ und 1 l Toluol wurde etwa 2 Stunden unter laufender Auskreisung des Reaktionswassers zum Sieden erhitzt. Nach Abkühlung des Reaktionsgemisches auf 25°C wurde das gebildete NaCl abgetrennt und mit 1 l Ethanol gewaschen. Toluol- und Ethanolphase wurden vereinigt und unter vermindertem Druck bei 40°C eingeengt. Der Rückstand wurde aus 0,2 l Ethanol unter Zusatz von Aktivkohle umkristallisiert.

Farblose Kristalle, Smp. 121—123°C; Ausbeute 60%.

### Beispiel 3

$A-NH-n-C_{12}H_{25}$

Eine Mischung aus 145 g (0,78 mol) Dodecylamin, 100 g (0,78 mol) A—OH, 1 g p-Toluolsulfonsäure und 50 ml Toluol wurde etwa 1 Stunde unter Auskreisung des Reaktionswassers zum Sieden erhitzt

und danach bei 60° C mit 500 ml n-Hexan versetzt. Beim weiteren Abkühlen schied sich das Verfahrensprodukt als gelblicher Kristallbrei ab. Die Ausbeute an der reinen, aus n-Hexan umkristallisierten Verbindung, die in Form farbloser Blättchen anfiel, betrug 81%; Smp. 79—81° C.

### Beispiele 4 bis 15

$A-NR^3-R^4$

Analog Beispiel 3 wurden verschiedene Aminoverbindungen $HNR^3-R^4$ mit A—OH umgesetzt. Die Ergebnisse sind der Tabelle 1 zu entnehmen.

Tabelle 1

| Beispiel | Verbindung $A-NR^3-R^4$ | Schmp. °C | Ausbeute % |
|---|---|---|---|
| 4 | $A-NH-CH_2-CH_2-OH$ | 164–165 | 63 |
| 5 | $A-NH-CH-COOH$<br>\|<br>$CH_3$ | 227–229 | 51 |
| 6 | $A-NH-CH_2-CH_2-COOH$ | 121–123 | 80 |
| 7 | $A-NH-CH_2-CH_2-COO-CH_3$ | 96–98 | 60 |
| 8 | $A-NH-CH_3$ | 175–178 | 48 |
| 9 | $A-NH-CH_2-CH-OH$<br>\|<br>$CH_3$ | 106–107 | 57 |
| 10 | $A-N-CH_2-CH_2-OH$<br>\|<br>$CH_3$ | flüssig | 64 |
| 11 | A—NH—n-hexyl | 101–102 | 70 |
| 12 | A—NH—n-tridecyl | 86–87 | 70 |
| 13 | A—NH—2-ethylhexyl | flüssig | 90 |
| 14 | A—N—2-ethylhexyl<br>\|<br>$CH_3$ | flüssig | 70 |
| 15 | $A-NH-(CH_2-CH_2-O)_2H$ | 85–89 | 52 |

### Beispiel 16

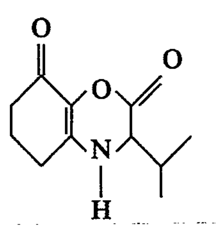

Eine Mischung aus 12,8 g (0,1 mol) A—OH, 12,9 g (0,11 mol) D,L-Valin, 50 ml Toluol und 1 g p-Toluolsulfonsäure wurde unter Auskreisen des Reaktionswassers etwa 2 Stunden lang zum Sieden erhitzt. In

dieser Zeit wurden 3,6 ml (0,2 mol) Wasser abgeschieden.

Danach wurde das Reaktionsgemisch abgekühlt, wobei das Verfahrensprodukt auskristallisierte. Die Kristallmasse wurde abgetrennt und aus Ethanol umkristallisiert. Die Ausbeute an dem reinen Lacton betrug 57%; Smp. 170—171° C, farblose Kristalle.

Beispiele 17—19

Analog Beispiel 16 wurden die Lactone gemäß Tabelle 2 aus den entsprechenden Ausgangsverbindungen hergestellt.

Tabelle 2

| Beispiel | Lacton | Schmp. °C | Ausbeute % |
|---|---|---|---|
| 17 | 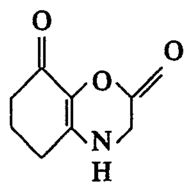 | 210–213 | 70 |
| 18 | 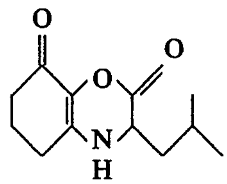 | 165–167 | 55 |
| 19 | 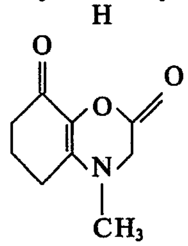 | 133–135 | 52 |

Stabilisierungstests

a) Stabilisierende Wirkung verschiedener Antioxidantien in Schweineschmalz

Schweineschmalz mit einem Gehalt von je 0,02 Gew.-% verschiedener Antioxidantien wurden dem sog. Peroxidtest (näheres s. z. B. Oil and Soap, Band 15, 1938, S. 184) unterworfen. Hierzu wurde das Probematerial solange unter Luftzutritt bei 80° C gelagert, bis es eine Peroxidzahl von 50 hatte.

Die Ergebnisse dieser Versuche sind in Tabelle 3 zusammengestellt.

Tabelle 3 Peroxidzahl (POZ) von Schweineschmelz

| Versuch | Antioxidans | Gemäß Beispiel Nr. | Dauer bis POZ = 50 Tage |
|---|---|---|---|
| Zum Vergleich | | | |
| 1 | ohne Zusatz | – | 1 |
| 2 | Ascorbinsäure | – | 1 |
| 3 | $\alpha$-Tocopherol | – | 1 |
| 4 | 2,3-Dihydroxycyclohex-2-en-1-on (A–OH) | – | 1 |
| 5 | 2/3-tert.-Butyl-4-hydroxyanisol(BHA) | – | 2 |
| 6 | 2/3-tert.-Butyl-4-hydroxytoluol (BHT) | – | 2 |
| Erfindungsgemäß | | | |
| 7 | A—NH—n-dodecyl | 3 | 4 |
| 8 | $A—NH—CH_2—COOH$ | 1 | 5 |
| 9 | A—NH—2-ethylhexyl | 14 | 7 |
| 10 | $A—NH—CH_2—CH_2—OH$ | 4 | 9,5 |
| 11 | $A—NH—CH_2—CH_2—COOH$ | 6 | 10,5 |
| 12 | $A—N(CH_3)—2$-ethylhexyl | 15 | 10,5 |
| 13 | $A—NH—CH_2—COO—C_2H_5$ | 2 | 10 |
| 14 | $A—NH—CH_3$ | 8 | 12 |
| 15 | 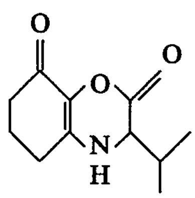 | 16 | 8 |
| 16 | 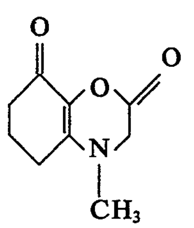 | 19 | 10 |

Wie man erkennt, sind die erfindungsgemäßen Antioxidantien sowohl anderen Reduktonverbindungen als auch sonstigen handelsüblichen Mitteln deutlich überlegen.

b) Stabilisierende Wirkung verschiedener Antioxidantien in $\beta$-Carotin-Trockenpulver

$\beta$-Carotinpulver wurde mit je 1,9 Gew.-% eines Antioxidans versetzt und wie üblich mit Gelatine als Überzugsmassse konfektioniert.

Die Pulver wurden mehrere Wochen lang bei 37° C unter Luftzutritt gelagert, wobei der $\beta$-Carotingehalt in bestimmten Zeitabständen ermittelt wurde. Die Ergebnisse sind in Tabelle 4 zusammengestellt.

Tabelle 4

| Versuch | Antioxidans | gemäß Beispiel Nr. | $\beta$-Carotingehalt in % des ursprünglichen Gehaltes nach Wochen 3 | 5 | 8 | 16 |
|---|---|---|---|---|---|---|
| zum Vergleich | | | | | | |
| 1 | ohne Zusatz | — | 14 | — | — | — |
| 2 | $\alpha$-Tocophenol | — | 86 | 80 | 70 | 70 |
| 3 | 3-N-Morpholino-5-methyl-2,3-dihydroxy-cyclo-hex-2-en-1-on | — | 72 | 64 | 60 | 50 |
| erfindungsgemäß | | | | | | |
| 4 | A—NH—n-dodecyl | 3 | 91 | 88 | 87 | 82 |
| 5 | A—N($CH_3$)—2-ethylhexyl | 14 | 88 | 85 | 80 | 80 |

Auch bei diesen Versuchen erwiesen sich die erfindungsgemäßen Mittel wesentlich besser als die Vergleichssubstanzen.

c) Stabilisierende Wirkung verschiedener Antioxidantien in Citranaxanthin-Trockenpulver

Citranaxanthin, das als Zusatz zu Legehennenfutter dient, wurde mit 2 Gew.-% des Antioxidans vermischt und mit Gelatine wie üblich zu einem Trockenpulver verarbeitet. Dieses Pulver wurde sodann in einer Konzentration von 20 ppm Wirkstoff mit handelsüblichen Legehennenfutter vermischt und 12 Wochen unter Luftzutritt gelagert, wobei der Citranaxanthingehalt alle 4 Wochen bestimmt wurde. Tabelle 5 zeigt die Ergebnisse, die klar zugunsten der erfindungsgemäßen Mittel sprechen.

Tabelle 5

| Versuch | Antioxidans | gemäß Beispiel Nr. | Citranaxanthingehalt in % des ursprünglichen Gehaltes und Wochen 4 | 8 | 12 |
|---|---|---|---|---|---|
| zum Vergleich | | | | | |
| 1 | ohne Zusatz | — | 8 | 5 | — |
| 2 | $\alpha$-Tocopherol | — | 13 | 5 | — |
| erfindungsgemäß | | | | | |
| 3 | A—NH—dodecyl | 3 | 37 | 36 | 26 |
| 4 | A—$NCH_3$—2-ethylhexyl | 14 | 23 | 20 | 15 |

**Patentansprüche**

1. Organische Materialien, gekennzeichnet durch einen Gehalt von 0,001—10 Gew.-% eines Aminoreduktons der allgemeinen Formel I

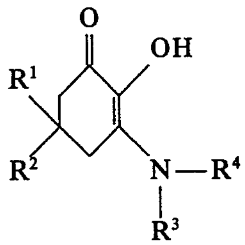

(I)

oder eines mineralsauren Salzes davon als Antioxidans, wobei die Substituenten in dieser Formel folgende Bedeutung haben:

$R^1$, $R^2$, $R^3$ H; die Methylgruppe oder die Ethylgruppe;

$R^4$ den an eine primäre Aminogruppe gebundenen Rest einer natürlichen α- oder β-Aminosäure oder einen $C_1—C_{10}$-Alkylester davon oder, falls es sich um eine α-Aminosäure handelt, um einen Rest, in welchem die Carboxylgruppe der Aminosäure und die 2-Hydroxylgruppe des Cyclohexenonrings ein Lacton bilden;
eine $C_1—C_{20}$-Alkyl- oder Alkenylgruppe, die eine oder mehrere Hydroxylgruppen, $C_1—C_4$-Alkoxygruppen oder $C_1—C_4$-Acyloxygruppen tragen kann, oder
einen Rest $—(CH_2—CH_2—O)_nH$, der mit einem $C_1—C_4$-Alkanol verethert oder mit einer $C_1—C_4$-Fettsäure verestert sein kann, wobei n für 2—10 steht.

2. Verwendung der Aminoreduktone I als Antioxidantien für die Stabilisierung von organischen Materialien.

3. Aminoreduktone der allgemeinen Formel Ia

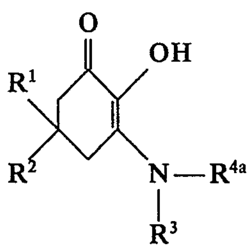

(Ia)

in welcher der Rest $R^{4a}$ mit Ausnahme der Methylgruppe die Bedeutung von $R^4$ hat.

4. Verfahren zur Herstellung der Aminoreduktone I, dadurch gekennzeichnet, daß man ein 2,3-Dihydroxy-cyclohex-2-en-1-on der allgemeinen Formel II

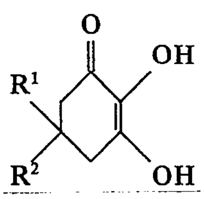

(II)

mit einer Aminoverbindung der allgemeinen Formel III

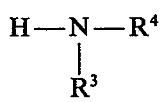

(III)

oder einem mineralsauren Salz davon umsetzt.

**Claims**

1. An organic material containing from 0,001 to 10% by weight of an aminoreductone of the general formula I

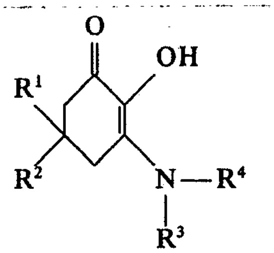

(I)

the substituents in this formula having the following meanings:

$R^1$, $R^2$ and $R^3$ H, methyl or ethyl; and
$R^4$ the radical, bonded to a primary amino group, of a naturally occurring $\alpha$- or $\beta$-aminoacid or a $C_1-C_{10}$-alkyl ester thereof or, in the case of an $\alpha$-aminoacid, a radical in which the carboxyl group of the aminoacid and the 2-hydroxyl group of the cyclohexenone ring form a lactone, or $C_1-C_{20}$-alkyl or -alkenyl, which may carry one or more hydroxyl, $C_1-C_4$-alkoxy or $C_1-C_4$-acyloxy groups, or is $-(CH_2-CH_2-O)_nH$, which may be etherified with a $C_1-C_4$-alkanol or esterified with a $C_1-C_4$-fatty acid, and where n is from 2 to 10,

or a mineral salt thereof, as an antioxidant.

2. The use of an aminoreductone I as an antioxidant for the stabilization of organic materials.

3. An aminoreductone of the general formula Ia

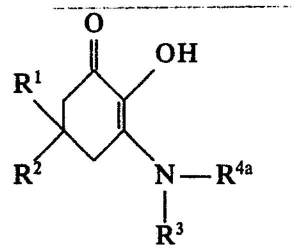

(Ia)

where $R^{4a}$ has the meanings of $R^4$, with the exception of methyl.

4. A process for the preparation of an aminoreductone I, wherein a 2,3-dihydroxy-cyclohex-2-en-1-one of the general formula II

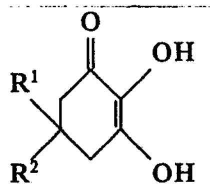

(II)

is reacted with an amino compound of the general formula III

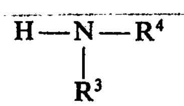

(III)

or a mineral acid salt thereof.

**Revendications**

1. Matières organiques, caractérisées par une teneur de 0,001 à 10% en poids d'une amino-réductone de la formule générale I

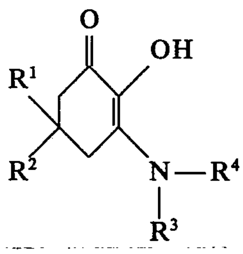

(I)

ou d'un sel d'un acide minéral d'un tel composé, comme anti-oxygène, les substituants, dans la formule ci-dessus, possédant les significations ci-après:

$R^1$, $R^2$ et $R^3$: H ou un radical méthyle ou éthyle;

$R^4$: le reste d'un $\alpha$- ou $\beta$-amino-acide naturel, lié à un groupe amino primaire, ou un ester d'alkyle en $C_1$ à $C_{10}$ de celui-ci ou, dans le cas d'un $\alpha$-amino-acide, un reste dans lequel le groupe carboxyle de l'acide aminé et le groupe 2-hydroxyle du noyau cyclohexénone forment une lactone, ou
un groupe alkyle ou alcényle en $C_1$ à $C_{20}$, pouvant porter un ou plusieurs groupes hydroxyle, alcoxy en $C_1$ à $C_4$ ou acyloxy en $C_1$ à $C_4$, ou
un groupe $-(CH_2-CH_2-O)_nH$, où n vaut de 2 à 10, pouvant être éthérifié par un alcanol en $C_1$ à $C_4$ ou estérifié par un acide gras en $C_1$ à $C_4$.

2. Utilisation des amino-réductones I comme anti-oxygènes pour la stabilisation de matières organiques.

3. Amino-réductones de la formule générale Ia

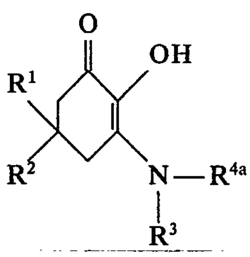

(Ia)

dans laquelle le substituant $R^{4a}$ possède les significations définies pour $R^4$, à l'exception du radical méthyle.

4. Procédé de préparation des amino-réductones I, caractérisé en ce que l'on fait réagir une 2,3-dihydroxy-cyclohex-2-ène-1-one de la formule générale II

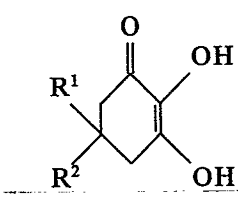

(II)

avec un dérivé d'amine de la formule générale III

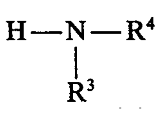

(III)

ou avec un sel d'un acide minéral d'un tel composé.